(19) Europäisches Patentamt
European Patent Office
Office européen des brevets

(11) **EP 3 985 678 A1**

(12) **EUROPEAN PATENT APPLICATION**

(43) Date of publication:
**20.04.2022 Bulletin 2022/16**

(21) Application number: **20201631.7**

(22) Date of filing: **13.10.2020**

(51) International Patent Classification (IPC):
*G16H 10/60* (2018.01)   *A61B 5/145* (2006.01)
*A61M 5/172* (2006.01)   *G16H 20/17* (2018.01)
*G16H 40/67* (2018.01)   *G16H 50/20* (2018.01)
*G16H 50/30* (2018.01)

(52) Cooperative Patent Classification (CPC):
**G16H 20/17; A61B 5/14503; A61B 5/14532;**
**A61B 5/4839; G16H 10/60; G16H 40/67;**
**G16H 50/20; G16H 50/30;** A61M 5/142;
A61M 5/1723; A61M 2005/14208; A61M 2205/3584;
A61M 2205/505

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Diabeloop**
**38028 Grenoble Cedex 1 (FR)**

(72) Inventor: **HUNEKER, Erik**
**92800 Puteaux (FR)**

(74) Representative: **Fidal Innovation**
**4-6 avenue d'Alsace**
**92400 Courbevoie (FR)**

(54) **COMPUTERIZED DEVICE FOR REPEATEDLY DETERMINING A COMMAND TO CONTROL THE INFUSION OF FLUID IN A DIABETIC PATIENT**

(57)   The computerized device repeatedly determines a command to control the infusion of fluid in a diabetic patient. It comprises:
- a processor (6) which repeatedly determines a value for a control parameter of a fluid infusion pump based on patient data stored in a database (27) and on a standard scheme,
- a user interface (7) which comprises a welcome screen (9) and at least a further screen displayable following an interaction of the patient with the welcome screen.

The welcome screen (9) comprises an actuable button (24) which enables to amend the standard scheme for a predefined temporary lapse of time into a temporary scheme.

[Fig. 2]

**Description**

FIELD OF THE INVENTION

The present invention relates to the field of computerized devices for the control of infusion of fluid into diabetic patients.

TECHNOLOGICAL BACKGROUND

More precisely, the invention relates to the control of infusion of fluid into diabetic patients.

In the field of diabetes, it is known to evaluate the concentration of blood glucose, and to inject a quantity of insulin as a function of the measured concentration. Recently, so-called "closed-loop" systems were developed, where a processor is programmed to evaluate a volume rate of insulin to be injected, based on patient data, and to control the injection of insulin based on this evaluation. In addition, the processor can be programmed to evaluate a volume of insulin to be injected in some special circumstances, in particular meals. The quantity can be injected to the patient, subject to the patient's approval. Such systems are also called "semi closed-loop", because of the necessary declaration by the patient of some of these special circumstances.

Determination of a correct quantity of insulin to be injected is very complex, for many different reasons. One reason is the very different biological nature of all patients. Another reason is the very large number of factors which affect the influence of the injected insulin to the blood glucose concentration. There are many other reasons.

Hence, the quantity or rate of insulin determined by the processor is necessarily a trade-off between various competing requirements. The processor will apply a more-or-less complex model to take into account this large number of factors.

Most often, the model can be parametered for the patient. This parametrization can be performed when the patient first uses the system, and is performed using medical knowledge by a doctor specialized in diabetes. Typically, the doctor will set from 2 to 20 parameters of the model to make the model specific to the patient. The patient-specific parametered model will be used by the controller to regulate the injection of insulin in a patient-specific way.

The patient themselves may also influence the result of the model. For example, the patient himself may define a glycemic target at a given value, which is the targeted value of glucose concentration at a given future time. The processor will determine the quantity or rate of insulin to be injected at the present time in order for the blood glucose at a given future time to be close to the defined target. Another option is to define a target range, rather than a target (so-called "control-to-range" algorithms). According to this definition, the processor will determine the quantity or rate of insulin to be injected at the present time in order for the blood glucose concentration at a given future time to be inside the defined target range.

Yet another option is, for the patient, to define a target profile, for example with various different targets or target ranges along with time. US 2017/332,952 describes an example of such schemes.

According to this document, the patient may temporarily wish to overrule the predefined profile, for example in order to temporarily reduce the risk of hyperglycemia. However, this requires a very complex parametrization scheme. This may cause errors for patients who are not extremely familiar with computers. Further, this parametrization may generate stress for the patient, which may adverse to the sought aim of temporarily reducing the risk of hypoglycemia.

The invention thus aims at democratizing the temporary target overrule function among diabetic patients.

SUMMARY OF THE INVENTION

Thus, the invention relates to a computerized device for repeatedly determining a command to control the infusion of fluid in a diabetic patient, wherein the computerized device comprises:

- a processor adapted to repeatedly determine a value for a control parameter of a fluid infusion pump based on patient data stored in a database and on a standard scheme,
- a user interface comprising a welcome screen and at least a further screen displayable following an interaction of the patient with the welcome screen,
  wherein the welcome screen comprises an actuable button adapted to, when actuated, amend the standard scheme for a predefined temporary lapse of time into a temporary scheme.

Thus, the invention makes it possible to easily define a temporary target overrule function. It will be very convenient for diabetic patients wishing not having to monitor for blood glucose for a temporary amount of time.

According to various aspects, one or more of the following features may be implemented.

According to some embodiments, the temporary scheme is adapted, upon application, to determine a value for the control parameter which reduces the risk of hypoglycemia compared to the value of the control parameter determined by the standard scheme.

According to some embodiments, the standard scheme is adapted to determine a value for the control parameter which maximizes the likelihood of a predicted blood glucose value predicted by the standard scheme to be close to a pre-defined target.

According to some embodiments, the temporary scheme defines a temporary target higher than the pre-defined target.

According to some embodiments, the standard scheme comprises an auto-learned model,

According to some embodiments, actuation of the actuable button triggers one or more of:

- a clock measuring the time spent from the time of the actuation, and a clock display displaying either that time, or the time remaining within the temporary period;

- a toggling of icon of the actuable button.

According to some embodiments, actuation of the actuable button within the temporary period causes one of:

- automatically stopping the temporary scheme,

- nothing,

- prolonging the temporary period.

According to some embodiments, the temporary scheme is not parametrizable by the patient.

According to some other aspects, the invention relates to a system for repeatedly determining a command to control the infusion of fluid in a diabetic patient, comprising a data acquisition system and such a computerized device, wherein data of the database was acquired by the data acquisition system.

According to some other aspects, the invention relates to a system for controlling the infusion of fluid in a diabetic patient comprising such a computerized device or such a system, and further comprising an active system receiving the value for the control parameter from the computerized device, and infusing fluid into the patient based on this value.

According to another aspect, the invention relates to a computer program for repeatedly determining a command to control the infusion of fluid in a diabetic patient, wherein the computer program is adapted, when run on a processor, to:

- cause the processor to repeatedly determine a value for a control parameter of a fluid infusion pump based on patient data stored in a database and on a standard scheme,
- cause the processor to display a welcome screen of a graphical user interface, wherein the graphical user interface further comprises at least a further screen displayable following an interaction of the patient with the welcome screen,

Wherein the welcome screen comprises an actuable button adapted to, when actuated, amend the standard scheme for a predefined temporary lapse of time into a temporary scheme.

According to another aspect, the invention relates to a fluid for delivery to a diabetic patient under a command determined by a processor of a computerized device which repeatedly determines a value for a control parameter of a fluid infusion pump based on patient data stored in a database and on a standard scheme, and a temporary scheme actuable for a predefined temporary lapse of time from an actuable button of a welcome screen of a graphical user interface further comprising a further screen displayable following an interaction of the patient with the welcome screen.

BRIEF DESCRIPTION OF THE DRAWINGS

Embodiments of the invention will be described below, in relation to the following drawings :

Fig. 1     shows a medical system.

Fig. 2     shows a welcome screen of the graphical user interface.

Fig. 3     shows a decision module.

Fig. 4     is a schematic view of a hand-held device.

On the drawings, the same reference signs show the same or similar objects.

In the present detailed description, and according to the WHO Expert Committee on Biological Standardization. one international unit of insulin (1 U) is defined as the "biological equivalent" of 34.7 micrograms ($\mu$g) pure crystalline insulin. This unit is the relevant unit for discussing a quantity of insulin to be infused to a patient, and can not be converted to the International System of Units, because the conversion would depend on which insulin is being used. For the sake of the disclosure of the present invention, it is important that quantities of insulin be expressed in a system meaningful for the invention, the readers and the scientific community.

DETAILED DESCRIPTION

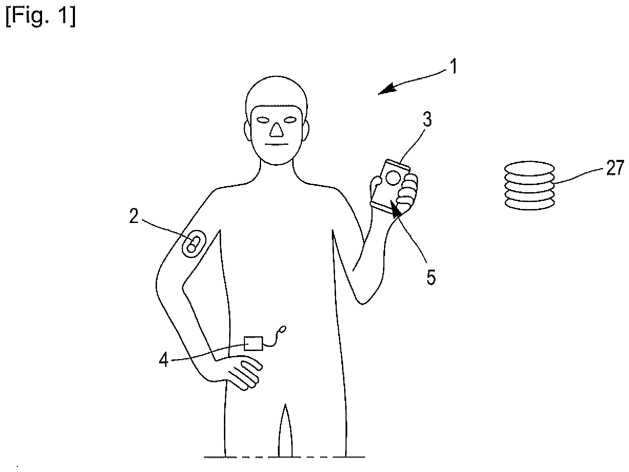

Figure 1 typically shows a medical system 1. The medical system 1 comprises a data acquisition system 2, a data processing system 3, and an active system 4.

The medical system 1 comprises a communication system adapted to rule communications between the various components of the system. In particular, in the present example, the communication system enables communication between the data acquisition system 2 and the data processing system 3, by which data acquired by the data acquisition system 2 can be transferred to the data processing system 3 for processing. The communication system enables communication between the data processing system 3 and the active system 4, by which control parameters of the active system 4 determined by the data processing system 3 can be transferred to the active system 4 to control the active system 4. The communication system is for example a wireless communication system operating according to one or more communication standards, such as Bluetooth.

The data acquisition system 2 acquires analyte patient data. The data acquisition system 2 may for example comprise a continuous glucose monitoring system, adapted to regularly estimate a concentration of blood glucose in the patient's blood. Various technologies are available. Importantly, this estimation is performed repeatedly, for example in the order of time of the minute. The acquired data may be pre-processed, and is stored in a database 27 accessible by the data processing system 3.

The data processing system 3 is part of a hand-held device 5. The data processing system 3 comprises a processor 6 which runs one or more computer programs in order to determine a control parameter for the active system 4.

The hand-held device 5 further comprises a user interface 7. A user interface 7 comprises a set of computer programs adapted to exchange information with the patient, i.e. to provide information to the patient and to get information from the patient. Typically, the user interface 7 is a graphical user interface which operates a tactile-sensitive display 8 of the hand-held device 5. The display 8 will be operative to provide visual information to the patient. Further, the touch screen will detect interactions of the patient with the hand-held device 5, and will register this information.

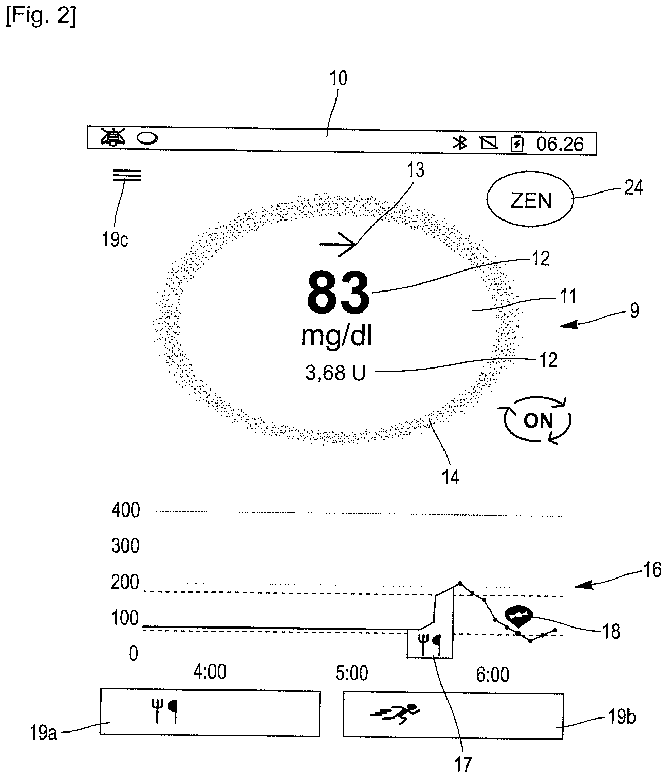

The user interface 7 comprises a welcome screen 9 which is shown on Figure 2. A welcome screen 9 is characterized by being the default screen which is displayed to the patient after the hand-held device 5 has been idle for some pre-defined time. The welcome screen 9 can be accessed potentially only after unlocking the graphical user interface and/or the patient logging in in the medical system. Unlocking the graphical user interface might be performed by detecting a pre-determined movement at a tactile surface of the hand-held device 5. Logging in might include some recognition of user identity, such as for example by recognition of a user-specific code or biometric identification. Further, all of, or a majority of, or at least one or a plurality of further screens of the graphical user interface 7 comprise a shortcut to the welcome screen controlling, when activated, the display of the welcome screen on the display 8.

The welcome screen may comprise one or more functionalities which are the most useful to the patient. In the present description, displaying an information is considered to be a functionality. However, other functionalities are also available, which are activated through an action of the patient.

An example of the welcome screen 9 will be described below, but other embodiments are also possible.

The welcome screen may comprise a menu window 10 which allows access to other functionalities. The menu window 10 is for example a narrow window at the top of the welcome screen. It may display operative parameters of the hand-held device 5, such as operationality of connection with remote devices, state of battery, current time of day, ... It may give access to operational parameters of the system through a further window which is accessible in any other suitable way such as, for example, pressing the menu window 10 and sliding downward.

The welcome screen 9 may comprise a blood glucose window 11 displaying a blood glucose value 12. For example, the blood glucose value 12 is the latest data measured by the data acquisition system 2 and transferred to the data processing system 3.

Further, or alternatively, the blood glucose window 11 may comprise an indicator of trend 13 of the blood glucose value. For example, the indicator of trend 13 is determined based on recent data measured by the data acquisition system 2 and transferred to the data processing system 3. Based on a plurality of past measurement points, it may indicate whether blood glucose is significantly going up, going down, or is stable, as shown in the present example.

A color indicator 14 might also be displayed in the blood glucose window. The color indicator 14 might indicate a normal situation or a potential alarm based on a calculation by the processor based, for example on past measured data, the blood glucose value, the trend, and/or other relevant data.

The blood glucose window 11 might also display an insulin on board value 15. The value of insulin on board might be determined by the data processing system based on a history of injected insulin, and on a model of insulin consumption by the patient. For example, this calculation is part of the model described below.

The welcome screen 9 may further comprise a time chart 16. The time chart 16 may for example display the evolution of one or more relevant parameter along time. The time will for example be provided in abscissa, and may cover a window of a predefined duration over the present time, covering one or both of past and future times. The parameter is provided as ordinate. It may comprise past blood glucose measurements and predicted future blood glucose values determined by the data processing system 3. It may further indicate a meal declared by the patient (represented by a meal icon 17) and a duration of the meal, represented by a greyed area under the blood glucose curve. It may further indicate a snack declared by the patient (represented by a snack icon 18).

The welcome screen 9 might also comprise menu buttons 19 enabling the patient, when actuated, to access further screens. For example, the welcome screen 9 comprises a meal declaration menu button 19a which, when actuated, causes the processor to display a meal declaration screen, where the patient has the possibility to enter relevant meal information such as time, duration, type of meal, number of CHOs, ... For example, the welcome screen 9 comprises a sport declaration menu button 19b which, when actuated, causes the processor to display a sport declaration screen, where the patient has the possibility to enter relevant sport information such as time, duration, type of sport, sport intensity, ... Alternately, the information could be provided through a vocal assistant or an extra dedicated sensor.

The welcome screen may further comprise a general menu button 19c giving access to other less frequent functionalities. For example, parametrization/personalization of the system by the patient or the doctor may be accessed through this button 19c. Actuating this general menu button 19c, the patient might for example access a menu enabling him to define a default blood glucose value (milligram of sugar per deciliter of patient blood) that the patient expects the system to target. This definition might use functions such as field selection, keyboard enabling to enter a number or scrolling menu in order to select a value in a pre-defined list, and validation. For example, a default value for this target value is 100 mg/dl.

The data-processing system 3 is adapted to determine a value of a control parameter of the active system 4. As described here, "a value of a control parameter" may be any kind of value of one or more control parameters, such as Boolean, integer, real values, look-up tables, function(s) of time or function(s) of another parameter, ... The control parameter may be a single control parameter, or a combined control parameter, for example a pair of independent control parameters.

The data-processing system 3 is adapted to repeatedly determine the value for the control parameter. For example, it comprises a clock scheduling repeated determination steps. Alternatively, the determination of the value for the control parameter is triggered by the reception of measurement data from the data acquisition system 2, or by other triggers, such as an activity declaration.

The determination is based on analyte data acquired by the data acquisition system 2, and on one or more computerized modules determining a value for the control parameters based on this data. The determination may alternatively or in addition be based on past determined values for the control parameter.

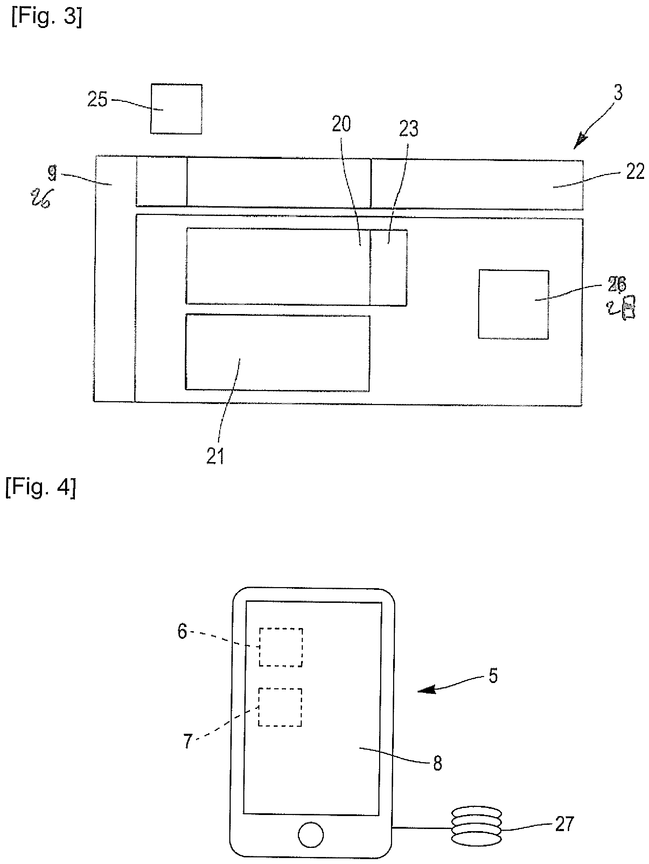

In the present example, the data-processing system operates four different modules according to a pre-established decision module, as shown on Figure 3. However, the present invention may be applied to any other kind of data-processing system which determines a control value for the active system 4 based on a data acquisition system 2 and one or more computerized modules.

According to one example, the data-processing module comprises a data-based control module. The data-based control module is designed to determine a value for the control parameter based on a data-driven model. The data-driven model is based on data representative of the relationship between the data measured by acquisition systems or declared by the patient and control parameter in other situations. According to one example, the control module 20 is a data-based module set up as a model predictive control module 20. The model predictive control module 20 comprises a biological model of the patient designed to model the interaction of injected insulin and blood glucose for the patient. This model is patient-specific, and depends on recent observation data of the patient (in terms of measured data of blood glucose concentration and associated insulin infusion control value) and may have been originally parametrized. Notably, the physiological parameters of the patient in the model may comprise glucose transfer rates between various physiological compartments, time to peak glucose absorption ingested by the patient, etc... The model predictive control module 20 takes into account a history of blood glucose of the patient and of injected insulin and other factors (declared meals, declared physical exercises, etc ...) to predict a likely future value for the blood glucose concentration of the patient depending on a value of the control parameter to be set. The control parameter is for example a rate of insulin to be injected. The value of the control parameter will be determined based on a pre-defined optimum. If, for example, the pre-defined optimum is to have a target value of 100 mg/dl for the predicted future value of the blood glucose concentration three hours after the calculation time, it will determine the value of the control parameter which will provide a predicted future value of the blood glucose three hours after the calculation time which will be closest to this target. "Closest" might be evaluated under a number of various definitions of a distance.

According to one example, the data-processing module comprises a so-called expert module 21, which is rule-based. For example, the expert module would apply pre-determined rules on available data. The available data taken into account by the expert module may comprise or be limited to the current and/or past level of blood glucose concentration, and the history of the control command. One example of such rules may comprise a look-up table defining a value of the control parameter depending on various situations. For example, the look-up table may comprise entries as to intervals for the current level of blood glucose and the current trend of blood glucose, and/or a short-term forecast of the blood glucose concentration, and selects a suitable equation to be applied, which will provide a value of the control parameter for each of the pre-defined interval entries.

According to one example, the data-processing module comprises a safety module 22, which will apply some specific pre-determined rules in case of determined safety risks. For example, the determined safety risk may be a risk of hypoglycemia, or a risk of hyperglycemia. The safety risk might be determined based on the available data, such as, for example, current level and/or trend of blood glucose, short-term forecast of the blood glucose concentration and/or long-term forecast of the blood glucose concentration.

According to one example, the data-processing module comprises an activity-handling module 28. Examples of activities handled by the activity-handling module include meals. The activity-handling module will apply some specific pre-determined rules depending on the activity.

According to one example, the data-processing module comprises one-or more auto-learning based modules. The auto-learning based module might be updated from time to time. This means that, at a given time, this module is not the originally-parametered module, but a module based on this originally-parametered module, which was updated in the meanwhile. The updating of the module may be based on historical data of the patient, and/or of other patients.

According to one example, the auto-learning module 25, which updates the auto-learning based module, is run less frequently than the determination of the value for the control parameter.

In particular, for example, the model predictive control module 20 might be a auto-learning based module.

At any given time, some of the above-described modules may be operated under a decision module 26.

For example, the safety module 22 is operated first. If the decision module 26 estimates that the safety risk is high, it will apply the safety module 22 to determine the value of the control parameter. If the decision module 26 estimates that the safety risk is low, it will move to the other modules. The values for "High" and "low" might be predetermined and/or parametrable , or even auto-learned as discussed above.

The value of the control parameter might be determined as a function of the value of the control parameters determined by the expert module 21 and the model predictive control module 20.

For example, the decision module 26 may comprise a confidence assessment module 23 which assesses a confidence in the value for the control parameter determined by the auto-learned module. For example, the confidence assessment module 23 applies the auto-learned module to past data, compares the result of applying the auto-learned module to past data with the actual measured data, and assesses the level of confidence in the auto-learned module based on this comparison. In such case, the value of the control parameter might be determined as a function of the value of the control parameters determined by the expert module 21 and the model predictive control module 20, and as a function of the confidence assessment module 23.

For example, the function rendered by the confidence assessment module 23 might be that, if the confidence is high, the auto-learned module is applied to determine the value for the control parameter and, if the confidence is low, the non-auto-learned module is applied to determine the value for the control parameter. "High" and "low" confidences might be based on pre-determined and/or parametrable thresholds. Alternatively, other functions might be used.

The above scheme is programmed to operate as default, and determines a value for a control parameter of the active system 4.

According to one embodiment, one of the modules operates in basal infusion mode. "Basal" is used to define a continuous mode of infusion of insulin where the control parameter of the insulin flow is determined as a value of flow (or rate) and, in the present example, is lower than 5 U/h.

Control of the basal rate by the Model-predicitive controller

One example of the modules operating in basal infusion mode is the model predictive control module 20. Under this module, the aggressiveness is taken into account according to the following formula:

$$Basal = min \ (Basal_{MAX}; \ Basal_{MPC}),$$

where:

$\text{Basal}_{MPC}$ is the value of the control parameter defined taking into account the physiological model of the patient, which does not take into account the aggressiveness,

$$\text{Basal}_{MAX} = \alpha_m \cdot f\,(\text{BasalRef}_M, \text{IS}, \underline{gl}, \text{Target})$$

where:

$\text{Basal}_{MAX}$ is the maximal value for the control parameter,

$\alpha_m$ is the aggressiveness factor,

$\text{BasalRef}_M$ is a pre-defined reference value for the infusion of basal insulin for the patient for this module,

IS is the sensitivity to insulin of the patient (for example, this parameter may be predefined),

Target is the above-defined target blood glucose concentration,

$\underline{gl}$ is the short-term predicted blood glucose concentration - Short-term corresponds for example to any predefined future time between 5 minutes and 1 hour from the instant time. and

f is a multiplicative factor determined as a function of the sensitivity to insulin of the patient IS, the target blood glucose concentration "Target", and the predicted blood glucose concentration $\underline{gl}$ at short term. The function f is a rising function of the predicted blood glucose concentration, for example continuous or stepwise. According to this function, $\text{Basal}_{MAX}$ will be raised if predicted short-time blood glucose concentration is high. This allows for more insulin to be injected if there is a prediction that blood glucose concentration will be higher in the short-term. This function thus enables to infuse less insulin if the patient has a current low blood glucose concentration, which reduces the risk of hypoglycemia for the patient. In addition, this function takes into account the lower sensibility of the patient to insulin by high blood glucose concentration.

Control of the basal rate by the expert module controller

According to one example, the expert module 21 operates in basal infusion mode. Under this module, the aggressiveness is taken into account according to the following formula:

$$\text{Basal} = \min\,(\text{Basal}_{MAX}; \text{Basal}_{EM}),$$

where:

$\text{Basal}_{EM}$ is the value of the control parameter defined using the expert module, which does not take into account the aggressiveness,

$$\text{Basal}_{MAX} = \alpha_e \cdot g\,(\text{BasalRef}_E, \text{IS}, \underline{gl}, \text{Target})$$

where:

$\text{Basal}_{MAX}$ is the maximal value of the control parameter,

$\alpha_e$ is the aggressiveness factor,

$\text{BasalRef}_E$ is a pre-defined reference value for the infusion of basal insulin for the patient for this model,

IS is the sensitivity to insulin of the patient (for example, this parameter may be predefined),

Target is the above-defined target blood glucose concentration,

$\underline{gl}$ is the short-term predicted blood glucose concentration - Short-term corresponds for example to any future time between 5 minutes and 1 hour from the instant time. and

g is a multiplicative factor defined as a function of $BasalRef_E$, of the current insulin sensitivity of the patient, of a predicted blood glucose concentration as defined above, and of the glycemic target.

In particular, in basal infusion mode, the aggressiveness of the expert module 21 and the aggressiveness of the model predictive control module 20 are equal to one another, which allows to apply the same level of aggressiveness in basal infusion mode, regardless of the module used: $\alpha_e = \alpha_m = \alpha$.

Control of the bolus by the Expert module controller

According to one example, the expert module 21 operates in bolus infusion mode. "Bolus" is used to define a mode of infusion of insulin where the control parameter of the insulin delivery is a quantity of insulin, rather than a flow of insulin. Under this module, the aggressiveness is taken into account according to the following formula:

$$Bolus = \alpha_b \cdot h,$$

where:

Bolus is the value of the control parameter,

$\alpha_b$ is the aggressiveness factor,

h is determined as the outcome of the expert module 21, which does not take into account the aggressiveness.

In particular, h may be determined by the expert module 21 based on one or more of the following parameters: a predicted blood glucose concentration value, a target or a target range, and a patient-specific value for the sensitivity to insulin. In particular, according to one embodiment, the patient-specific value for the sensitivity to insulin s is a multiplicative factor of an outcome $h_0$ of the expert module 21 which does not take into account the sensitivity to insulin of the patient, whereby h may be written $h = s \cdot h_0$. In this case, Bolus may be written as

$$Bolus = \alpha_b \cdot s \cdot h_0,$$

whereby aggressiveness might be taken into account directly by modifying a patient-specific value for a factor $s_b$ of the sensitivity to insulin $s_b = \alpha_b \cdot s$.

In particular, the aggressiveness factor in bolus infusion mode $\alpha_b$ might be different from the aggressiveness value in basal infusion mode or, in case of various aggressiveness values in basal infusion mode, from one or more or all of the various aggressiveness values in basal infusion mode. Because a patient might not be willing to experience a similar behavior in basal infusion mode and in bolus infusion mode, setting different values for the aggressiveness in the various modes may offer a better control to the patient. However, in a different variant, the aggressiveness factor in bolus infusion mode $\alpha_b$ might be equal to the aggressiveness value in one of the basal infusion modes. This would make the control simpler for the patient.

Control of the delivery by the activity-handling module controller

According to another example, the activity-handling module 28 will now be described. In particular, the activity-handling module will be described in the context of a meal-handling module. However, the activity-handling module may handle other activities, such as physical exercise, sleep, ...

In the present example, the meal-handling module will be described as based on a declaratory module. Under a declaratory module, the patient is provided with an interface enabling him/her to provide the meal-handling module with information regarding a meal. Information may include various information enabling the meal-handling module to take into account the meal for the determination of insulin to be infused, Information may include one or more of:

Occurrence of a meal (Y/N ?),

Type of meal (Breakfast, lunch, dinner, snack, ...?),

Time and/or duration of the meal (either past, present or predicted future),

Nutritive information (estimated amount of CHO, ...).

The meal-handling module is not necessarily a declaratory module. It may be based on analysis of a blood glucose concentration curve.

The meal-handling module may determine a quantity of insulin to be infused to take into account the meal. This determination comes separate from the determination described above by the decision module 9, which is performed not taking into account any meal information.

In particular, the meal-handling module may operate in bolus mode. Under this module, the aggressiveness is taken into account according to the following formula:

$$\mathrm{MealBolus} = \alpha_{mb} \cdot i,$$

where:

MealBolus is the value of the control parameter,

$\alpha_{mb}$ is the aggressiveness factor, which is a real parameter, and may depend on the type of meal (i.e. a declaration by the patient of a type of meal, as discussed above), and of the declared quantity of ingested carbohydrates, and/or the detected blood glucose concentration.

i is determined as the outcome of the meal-handling module, based at least on the target, which does not take into account the aggressiveness.

According to the present exemplary embodiment, $\alpha_{mb}$ might be either positive, null or negative. The present scheme ensures that a meal bolus will often be determined by the meal-handling module, except in exceptional circumstances where $\alpha_{mb} \cdot i$ will be negative and be larger than MB in absolute value. In particular, i will take into account the meal information.

According to this specific module, it may be provided that the amount of Meal Bolus is prompted to the patient using the user interface, and the user exerts a validation process in order to trigger injection of the insulin. The validation process may include the user amending the calculated MealBolus using the user interface.

Further, the meal-handling module may determine a schedule for distribution of the meal bolus. This determination may be based on the above input parameters. For example, the schedule may comprise a distribution of the meal bolus in two parts spaced in time. The volume of each part may be also determined by the meal-handling module. For example, the meal-handling module would determine a 60%-40% two-part distribution of the whole bolus.

The data processing system 3 regularly communicates with the active system 4 to send the determined value of the control parameter to the active system 4. The active system 4 receives the value of the control parameter, and controls its operation based on this value.

For example, if the determined value of the control parameter is to set an instantaneous flow debit of insulin to 1 U/h ml/min, it operates the pump at this debit.

This operation could be applied until a new command is received from the data processing system, or for a pre-defined period of time.

For example, if the determined value of the control parameter is to deliver an instantaneous quantity of insulin of 10 U, it operates the pump to deliver this quantity.

As was discussed above, the value for the control parameter may be determined in order to optimize a particular cost function.

In the example provided above, the cost function might be minimized if the predicted glycemic value at a future instant of time is as close as possible to a target value.

Other cost functions might be implemented.

For example, the cost function might be minimized if the predicted glycemic value curve at a future time interval is as close as possible to a target curve.

The present invention provides for a temporary mode. The temporary mode is accessible through a button 24 actuable from the welcome screen 9. A single actuation of the button 24 actuates the temporary mode. An icon might be modified on the welcome screen 9 to indicate that the temporary mode was actuated. The temporary mode is actuated for a pre-defined period of time, for example for a pre-defined period of time comprised between one hour and five hours, typically between two hours and four hours. During the temporary period, the data processing system 3 operates a temporary scheme for the determination of the value of the control parameter. At the end of the temporary mode, the data processing system 3 automatically operates the standard determination scheme described above. It should be noted that the standard determination scheme is called "standard" here even though it is patient-specific, and though some components of these scheme might be updated from time to time, such as through using a auto-learning process.

The temporary mode might be programmed to reduce the risk of hypoglycemia of the patient. In particular, the temporary mode might provide a risk of hypoglycemia for the patient, which is lower than the standard mode, for a period of time starting from the time where the temporary mode is actuated to a future time. The future time is for example the end of the pre-defined period of time for the temporary mode. However, it may be a later time if, for example, the temporary period lasts until a given point of time, the determination of the value of the control parameter just before this point of time of the end of the temporary period will provide a reduced risk of hypoglycemia for a period of time which goes beyond the end of the temporary period.

The risk of hypoglycemia might be determined according to various ways. According to an example, the risk of hypoglycemia would be determined as a proportion of time during which the predicted glycemic value is below a pre-defined threshold, during an upcoming time window of pre-defined time duration. According to another example, the risk of hypoglycemia would be determined as an area between the curve for the predicted glycemic value and a pre-defined curve over time, for example a constant at a given pre-determined threshold over time. Other criteria might also be defined.

There are various ways for the temporary mode to provide a lower risk of hypoglycemia.

One example is to set a higher target value for the blood glucose concentration. By setting a higher target value than that of the default model, it is expected that the risk of hypoglycemia will be lower.

Another example is to set a higher threshold for the value of blood glucose concentration which controls using the hypoglycemia-adverse safety module 22.

Yet another example is to modify the aggressiveness parameter of one or more of the above modules.

According to one example, the time remaining in the temporary mode until the end of the temporary mode is measured by a clock. The welcome screen 9 comprises a clock window where the time remaining in the temporary mode is displayed.

According to one embodiment, the patient is prevented from actuating the button 19c starting the temporary mode if this temporary mode is already on. Simply, in this case, actuating the button 19c has no effect.

According to another example, actuating the button 19c for the temporary mode while the temporary mode is already on will automatically stop the temporary mode. In this case, the temporary mode will automatically stop, and the data-processing system 3 will immediately operate again in default mode, and the icon might be modified to indicate that the temporary mode is available again.

According to yet another example, actuating the button 19c for the temporary mode while the temporary mode is already on will cause the temporary mode to last for the predefined period of time from the time where the button is actuated again. The clock would be re-started, and the clock window amended. For example, if the temporary mode has been going on already for two hours, and the patient actuates the temporary mode button 19c again, the temporary mode will go one for the pre-defined time from this point of time, resulting in a longer overall time in the temporary mode.

According to an example, the temporary mode is not parametrizable by the patient. This means that the modification that the temporary mode exerts with respect to the standard mode is built-in, and the patient can not access to any menu to determine some specific parameters of this modification. The patient is not allowed to determine any specific parameters, such as any temporary glycemia target profile, temporary aggressiveness, or even duration. There is no interface enabling access to these parameters.

The above example is a three component system where an intermediate device comprises both the data processing system 3 and the user interface 7. However, other embodiments could be possible, such as, for example, having the data processing system 3 and the user interface 7 in different devices, and/or incorporating one or the other in the data acquisition system 2 or in the active system 4.

The above description is based on data acquired by a data acquisition system 2. The data acquisition system may include one single blood glucose sensor. Alternatively, it may include more than one blood glucose sensors, located in various locations of the patient's body. Further, the data acquisition system may additionally comprise further data sensors, to acquire other relevant data of the patient. The data processing system 3 may also take into account not patient specific data. The data processing system 3 is adapted to determine the value for the control parameter based on the data acquired by the data acquisition system 2.

Even though the system above was described in relation to the infusion of insulin, other embodiments are foreseen, such as, for example, a controlling the infusion of insulin and of a counter-agent to insulin such as glucagon.

References

Medical system 1

Data acquisition system 2

Data processing system 3

Active system 4

Hand-held device 5

Processor 6

User interface 7

Display 8

welcome screen 9

menu window 10

blood glucose window 11

blood glucose value 12

indicator of trend 13

Color indicator 14

Insulin on board value 15

Time chart 16

Meal icon 17

Snack icon 18

Menu buttons 19

Model predictive control module 20

Expert module 21

Safety module 22

Confidence assessment module 23

Button 24

Auto-learning module 25

decision module 26

database 27

Activity handling module 28

**Claims**

1. A computerized device for repeatedly determining a command to control the infusion of fluid in a diabetic patient, wherein the computerized device comprises:

   - a processor (6) adapted to repeatedly determine a value for a control parameter of a fluid infusion pump based on patient data stored in a database (27) and on a standard scheme,
   - a user interface (7) comprising a welcome screen (9) and at least a further screen displayable following an interaction of the patient with the welcome screen,
   Wherein the welcome screen (9) comprises an actuable button (24) adapted to, when actuated, amend the standard scheme for a predefined temporary lapse of time into a temporary scheme.

2. A computerized device according to claim 1, wherein the temporary scheme is adapted, upon application, to determine a value for the control parameter which reduces the risk of hypoglycemia compared to the value of the control parameter determined by the standard scheme.

3. A computerized device according to claim 1 or 2, wherein the standard scheme is adapted to determine a value for the control parameter which maximizes the likelihood of a predicted blood glucose value predicted by the standard scheme to be close to a pre-defined target.

4. A computerized device according to claim 3, wherein the temporary scheme defines a temporary target higher than the pre-defined target.

5. A computerized device according to any of claims 1 to 4, wherein the standard scheme comprises an auto-learned model.

6. A computerized device according to any of claims 1 to 5, wherein actuation of the actuable button triggers one or more of:

   - a clock measuring the time spent from the time of the actuation, and a clock display displaying either that time, or the time remaining within the temporary period;
   - a toggling of icon of the actuable button.

7. A computerized device according to any of claims 1 to 6, wherein actuation of the actuable button within the temporary period causes one of:

   - automatically stopping the temporary scheme,
   - nothing,
   - prolonging the temporary period.

8. A computerized device according to any of claims 1 to 7, wherein the temporary scheme is not parametrizable by the patient.

9. A system for repeatedly determining a command to control the infusion of fluid in a diabetic patient, comprising a data acquisition system and a computerized device according to any of claims 1 to 8, wherein data of the database (27) was acquired by the data acquisition system.

10. A system for controlling the infusion of fluid in a diabetic patient comprising a computerized device according to any of claims 1 to 8 or a system according to claim 9, and further comprising an active system (4) receiving the value for the control parameter from the computerized device, and infusing fluid into the patient based on this value.

11. A computer program for repeatedly determining a command to control the infusion of fluid in a diabetic patient, wherein the computer program is adapted, when run on a processor, to:

   - cause the processor to repeatedly determine a value for a control parameter of a fluid infusion pump based on patient data stored in a database (27) and on a standard scheme,
   - cause the processor to display a welcome screen (9) of a graphical user interface (7), wherein the graphical user interface (7) further comprises at least a further screen displayable following an interaction of the patient with the welcome screen (9),

Wherein the welcome screen (9) comprises an actuable button (24) adapted to, when actuated, amend the standard scheme for a predefined temporary lapse of time into a temporary scheme.

12. Fluid for delivery to a diabetic patient under a command determined by a processor of a computerized device which repeatedly determines a value for a control parameter of a fluid infusion pump based on patient data stored in a database (27) and on a standard scheme, and a temporary scheme actuable for a predefined temporary lapse of time from an actuable button (24) of a welcome screen (9) of a graphical user interface (7) further comprising a further screen displayable following an interaction of the patient with the welcome screen (9).

[Fig. 1]

[Fig. 2]

[Fig. 3]

[Fig. 4]

Europäisches
Patentamt

European
Patent Office

Office européen
des brevets

## EUROPEAN SEARCH REPORT

Application Number

EP 20 20 1631

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/005633 A1 (FINAN DANIEL [US]) 2 January 2014 (2014-01-02) * abstract; figures 1,2, 8 * * paragraph [0054] - paragraph [0064] * * paragraph [0076] - paragraph [0084] * * paragraphs [0126], [0139] - paragraph [0140] * * paragraph [0159] - paragraphs [0162], [0170] * | 1-12 | INV. G16H10/60 A61B5/145 A61M5/172 G16H20/17 G16H40/67 G16H50/20 G16H50/30 |
| A | US 2018/169334 A1 (GROSMAN BENYAMIN [US] ET AL) 21 June 2018 (2018-06-21) * the whole document * | 1-12 | |
| A | US 2015/190098 A1 (PATEK STEPHEN D [US] ET AL) 9 July 2015 (2015-07-09) * the whole document * | 1-12 | |
| A | Feuerstein-Simon Carly ET AL: "Use of a Smartphone Application to Reduce Hypoglycemia in Type 1 Diabetes: A Pilot Study", Journal of diabetes science and technology, 1 November 2018 (2018-11-01), pages 1192-1199, XP055789689, Los Angeles, CA DOI: 10.1177/1932296817749859 Retrieved from the Internet: URL:https://www.ncbi.nlm.nih.gov/pmc/articles/PMC6232731/pdf/10.1177_1932296817749859.pdf [retrieved on 2021-03-24] * the whole document * | 1-12 | TECHNICAL FIELDS SEARCHED (IPC) G16H A61B A61M |

The present search report has been drawn up for all claims

| Place of search | Date of completion of the search | Examiner |
|---|---|---|
| Munich | 28 March 2021 | Kutzarova, Iskrena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 1631

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2021

| Patent document cited in search report | Publication date | Patent family member(s) | | Publication date |
|---|---|---|---|---|
| US 2014005633 A1 | 02-01-2014 | AU | 2013280898 A1 | 12-02-2015 |
| | | BR | 112014032912 A2 | 27-06-2017 |
| | | CA | 2877734 A1 | 03-01-2014 |
| | | CN | 104394907 A | 04-03-2015 |
| | | EP | 2866855 A1 | 06-05-2015 |
| | | HK | 1209670 A1 | 08-04-2016 |
| | | JP | 6262222 B2 | 17-01-2018 |
| | | JP | 2015521902 A | 03-08-2015 |
| | | KR | 20150027245 A | 11-03-2015 |
| | | RU | 2015102842 A | 20-08-2016 |
| | | TW | 201408343 A | 01-03-2014 |
| | | US | 2014005633 A1 | 02-01-2014 |
| | | WO | 2014004159 A1 | 03-01-2014 |
| US 2018169334 A1 | 21-06-2018 | CA | 3046100 A1 | 28-06-2018 |
| | | CN | 110191731 A | 30-08-2019 |
| | | EP | 3558422 A1 | 30-10-2019 |
| | | US | 2018169332 A1 | 21-06-2018 |
| | | US | 2018169333 A1 | 21-06-2018 |
| | | US | 2018169334 A1 | 21-06-2018 |
| | | US | 2018174675 A1 | 21-06-2018 |
| | | US | 2021043297 A1 | 11-02-2021 |
| | | US | 2021043298 A1 | 11-02-2021 |
| | | US | 2021043299 A1 | 11-02-2021 |
| | | US | 2021043300 A1 | 11-02-2021 |
| | | WO | 2018119150 A1 | 28-06-2018 |
| US 2015190098 A1 | 09-07-2015 | AU | 2012300331 A1 | 17-04-2014 |
| | | BR | 112014004529 A2 | 13-06-2017 |
| | | CA | 2846854 A1 | 07-03-2013 |
| | | CN | 103907116 A | 02-07-2014 |
| | | CN | 106326651 A | 11-01-2017 |
| | | EP | 2748747 A1 | 02-07-2014 |
| | | EP | 3799073 A1 | 31-03-2021 |
| | | JP | 2014534483 A | 18-12-2014 |
| | | RU | 2014111290 A | 27-10-2015 |
| | | US | 2015190098 A1 | 09-07-2015 |
| | | WO | 2013032965 A1 | 07-03-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017332952 A